# EUROPEAN PATENT SPECIFICATION

(11) **EP 1 556 359 B1**
(45) Date of publication and mention of the grant of the patent: **18.06.2008**
(21) Application number: 03799023.1
(22) Date of filing: 24.09.2003
(51) Int. Cl.: C07D 231/18, A61K 31/4166

(54) **PYRAZOLE DERIVATIVES AND THEIR USE AS THERAPEUTIC AGENTS FOR HIV MEDIATED DISEASES**
PYRAZOLDERIVATE UND DEREN VERWENDUNG ALS THERAPEUTISCHE MITTEL FÜR DURCH HIV VERMITTELTE KRANKHEITEN
DERIVES DE PYRAZOLE ET UTILISATION DE CEUX-CI COMME AGENTS THERAPEUTIQUES DESTINES AUX MALADIES INDUITES PAR LE VIH

(30) Priority: 07.10.2002 GB 0223234
(43) Date of publication of application: 27.07.2005
(73) Proprietor: Pfizer Limited, Sandwich, Kent CT13 9NJ (GB); PFIZER INC., New York, N.Y. 10017 (US)
(72) Inventor: EDWARDS, Paul, John, Pfizer Global R. and D., Sandwich, Kent, CT13 9NJ (GB); JONES, Lyn, Howard, Pfizer Global R. and D., Sandwich, Kent CT13 9NJ (GB); MOWBRAY, Charles, Eric, Pfizer Global R. and D., Sandwich, Kent CT13 9NJ (GB); STUPPLE, Paul, Anthony, Pfizer Global R. and D., Sandwich, Kent CT13 9NJ (GB); TRAN, Isabelle, Pfizer Global R. and D., Sandwich, Kent CT13 9NJ (GB)
(74) Representative: Swarbrick, Terry Mark
(86) International application number: PCT/IB2003/004214
(87) International publication number: WO 2004/031156

(56) References cited:
- WO-A-02/04424
- WO-A-02/30907
- WO-A-02/085860
- DATABASE CA [Online] CHEMICAL ABSTRACTS SERVICE, COLUMBUS, OHIO, US; STANOVNIK, B. ET AL: "Product class 1: pyrazoles" retrieved from STN Database accession no. 139:214344 XP002265853 & SCIENCE OF SYNTHESIS (2002), 12, 15-225,

## Description

This invention relates to pyrazole derivatives, to their use in medicine, to compositions containing them, to processes for their preparation and to intermediates used in such processes.

Reverse transcriptase is implicated in the Infectious lifecycle of Human Immunodeficiency Virus (HIV). Compounds which interfere with the function of this enzyme have shown utility in the treatment of conditions caused by HIV and genetically related retroviruses, such as Acquired immune Deficiency Syndrome (AIDS). There is a constant need to provide new and better modulators, especially inhibitors, of HIV reverse transcriptase, since the virus is able to mutate, becoming resistant to the effects of known modulators.

Antiviral activity is ascribed to a class of N(hydroxyethyl)pyrazole derivatives in US patent number 3,303,200. A number of pyrazoles are disclosed as reverse transcriptase inhibitors, including: a class of N-phenylpyrazoles (J. Med. Chem., 2000, 43, 1034); a class of C and S linked aryl pyrazoles (WO02/04424); and a class of O and S linked aryl pyrazoles, the O and S aryl link being adjacent to the nitrogen atom (WO02/30907).

According to the present invention there is provided a compound of formula (I) 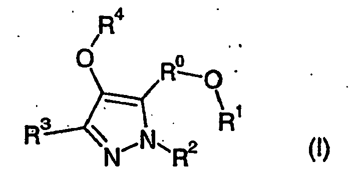 or a pharmaceutically acceptable salt or solvate thereof, wherein:
R⁰ is absent or C₁-C₆ alkylene;
R¹ is phenyl substituted by -SO_{y}R⁵, -(C₁-C₆ alkylene)-SO_{y}R⁵, -SO_{y}CF₃, -(C₁-C₆ alkylene)-SO_{y}CF₃, -CO₂R⁵, -(C₀-C₆ alkylene)-CO₂R⁵, OCF₃, a five or six-membered aromatic heterocyclic group containing (i) from 1 to 4 nitrogen heteroatom(s) or (ii) 1 or 2 nitrogen heteroatom(s) and 1 oxygen or 1 sulphur heteroatom (said heterocyclic group being optionally substituted by halo, oxo, -CN, -COR⁵, -CO₂R⁵, -CONR⁵R⁵, -SO_{y}R⁵, -SO_{y}CF₃, -SO₂NR⁵R⁵, -NR⁵SO₂R⁵, -OR⁵, -OCF₃, -NR⁵R⁵, -(C₁-C₆ alkylene)-NR⁵R⁵, C₁-C₆ alkyl, fluoro(C₁-C₆)alkyl or C₃-C₇ cycloalkyl), said phenyl being optionally additionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.
R² is H, C₁-C₆ alkyl, C₃-C₆ alkenyl, C₃-C₆ alkynyl, C₃-C₇ cycloalkyl, C₃-C₇ cycloalkenyl, phenyl, benzyl, R⁸ or R⁹, said C₁-C₆ alkyl, C₃-C₇ cycloalkyl, phenyl and benzyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ or R⁹;
R³ is H, C₁-C₆ alkyl, C₃-C₇ cycloalkyl, phenyl, benzyl, halo, -CN, -OR⁷, -CO₂R⁵, -CONR⁵R⁵, R⁸ or R⁹, said C₁-C₆ alkyl, C₃-C₇ cycloalkyl, phenyl and benzyl being optionally substituted by halo, -CN, -CR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ or R⁹;
R⁴ is phenyl, naphthyl or pyridyl, each being optionally substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹¹, SOₓR⁶, O-(C₁-C₆ alkylene)-CONR⁵R⁵, O-(C₁-C₆ alkylene)-NR⁵R⁵, or O-(C₁-C₆ alkylene)-OR⁶;
   each R⁵ is independently either H, C₁-C₆ alkyl or C₃-C₇ cycloalkyl or, when two R⁶ groups are attached to the same nitrogen atom, those two groups taken together with the nitrogen atom to which they are attached represent azetidinyl, pyrrolidinyl, piperidinyl, homopiperidinyl, piperazinyl, homopiperazinyl or morpholinyl, said azetidinyl, pyrrolidinyl, piperidinyl, homopiperidinyl, piperazinyl, homopiperazinyl and morpholinyl being optionally substituted by C₁-C₆ alkyl or C₃-C₇ cycloalkyl;
   each R⁶ is independently either H, C₁-C₆ alkyl or C₃-C₇ cycloalkyl;
   R⁷ is C₁-C₆ alkyl or C₃-C₇ cycloalkyl;
   R⁸ is a five or six-membered, aromatic heterocyclic group containing (i) from 1 to 4 nitrogen heteroatom(s) or (ii) 1 or 2 nitrogen heteroatom(s) and 1 oxygen or 1 sulphur heteroatom or (iii) 1 or 2 oxygen or sulphur heteroatom(s), said heterocyclic group being optionally substituted by halo, oxo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl;
   R⁹ is a four to seven-membered, saturated or partially unsaturated heterocyclic group containing (i) 1 or 2 nitrogen heteroatom(s) or (ii) 1 nitrogen heteroatom and 1 oxygen or 1 sulphur heteroatom or (iii) 1 oxygen or sulphur heteroatom, said heterocyclic group being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN;
   R¹⁰ is C₁-C₆ alkyl substituted by R⁸, R⁹, -OR⁵, -CONR⁵R⁵, -NR⁵COR⁵ or -NR⁵R⁵;
   R¹¹ is phenyl optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl; and
   x and y are independently 0, 1 or 2.

In the above definitions, halo means fluoro, chloro, bromo or iodo. Unless otherwise stated, alkyl, alkenyl, alkynyl, alkylene and alkoxy groups containing the requisite number of carbon atoms can be unbranched or branched chain. Examples of alkyl include methyl, ethyl, n-propyl, i-propyl, n-butyl, i-butyl, sec-butyl and t-butyl. Examples of alkenyl include ethenyl, propen-1-yl, propen-2-yl, propen-3-yl, 1-buten-1-yl, 1-buten-2-yl, 1-buten-3-yl, 1-buten-4-yl, 2-buten-1-yl, 2-buten-2-yl, 2-methylpropen-1-yl or 2-methylpropen-3-yl. Examples of alkynyl include ethynyl, propyn-1-yl, propyn-3-yl, 1-butyn-1-yl, 1-butyn-3-yl, 1-butyn-4-yl, 2-butyn-1-yl. Examples of alkylene include methylene, 1,1-ethylene, 1,2-ethylene, 1,1-propylene, 1,2-propyfene, 2,2-propylene and 1,3-propylene. Examples of alkoxy include methoxy, ethoxy, n-propoxy, i-propoxy, n-butoxy, i-butoxy, sec-butoxy and t-butoxy. Examples of cycloalkyl include cyclopropyl, cyclobutyl, cyclopentyl, cyclohexyl and cycloheptyl. Where a heterocyclic group R¹, R⁸ or R⁹ is attached to an oxygen, sulphur or nitrogen heteroatom the heterocyclic group R¹, R⁸ or R⁹ must be linked through a ring carbon atom.

The pharmaceutically acceptable salts of the compounds of formula (I) include the acid addition and the base salts thereof.

Suitable acid addition salts are formed from acids which form non-toxic salts and examples are the hydrochloride, hydrobromide, hydroiodide, chloride, bromide, iodide, sulphate, bisulphate, nitrate, phosphate, hydrogen phosphate, acetate, fumarate, pamoate, aspartate, besylate, carbonate, bicarbonate/, camsylate, D and L-lactate, D and L-tartrate, esylate, mesylate, malonate, orotate, gluceptate, methylsulphate, stearate, glucuronate, 2-napsylate, tosylate, hibenzate, nicotinate, isethionate, malate, maleate, citrate, gluconate, succinate, saccharate, benzoate, esylate, and pamoate salts.

Suitable base salts are formed from bases which form non-toxic salts and examples are the sodium, potassium, aluminium, calcium, magnesium, zinc, choline, diolamine, olamine, arginine, glycine, tromethamine, benzathine, lysine, meglumine and diethylamine salts.

For reviews on suitable salts see Berge et al, J. Pharm. Sci., 66, 1-19, 1977 and Bighley et al, Encyclopedia of Pharmaceutical Technology, Marcel Dekker Inc, New York, 1996, Vol 13, pp453-497

The pharmaceutically acceptable solvates of the compounds of formula (I) include the hydrates thereof.

The compound of formula (I) may be modified to provide pharmaceutically acceptable derivatives thereof at any of the functional groups in the compound. Examples of such derivatives are described in: Drugs of Today, Volume 19, Number 9, 1983, pp 499 - 538; Topics in Chemistry, Chapter 31, pp 306 - 316; and in "Design of Prodrugs" by H. Bundgaard, Elsevier, 1985, Chapter 1 and include: esters, carbonate esters, hemi-esters, phosphate esters, nitro esters, sulfate esters, sulfoxides, amides, sulphonamides, carbamates, azo-compounds, phosphamides, glycosides, ethers, acetals and ketals.

The invention encompasses all isomers of the compound of formula (I) and pharmaceutically acceptable salts or solvates thereof, including all geometric, tautomeric and optical forms, and mixtures thereof (e.g. racemic mixtures).

Separation of diastereoisomers may be achieved by conventional techniques, e.g. by fractional crystallisation, chromatography or high performance liquid chromatography (HPLC) of a stereoisomeric mixture of compounds. An individual enantiomer of a compound may also be prepared from a corresponding optically pure intermediate or by resolution, such as by HPLC of the corresponding racemate using a suitable chiral support, or by fractional crystallisation of the diastereoisomeric salts formed by reaction of the corresponding racemate with a suitable optically active acid or base, as appropriate.

The compound of formula (I) and pharmaceutically acceptable salts or solvates thereof may have the ability to crystallize in more than one form, a characteristic known as polymorphism, and all such polymorphic forms ("potymorphs") are encompassed within the scope of the invention. Polymorphism generally can occur as a response to changes in temperature or pressure or both, and can also result from variations in the crystallization process. Polymorphs can be distinguished by various physical characteristics, and typically the x-ray diffraction patterns, solubility behaviour, and melting point of the compound are used to distinguish polymorphs.

Compounds of formula (I), pharmaceutically acceptable salts or solvates thereof, isomers thereof, and polymorphs thereof, are hereinafter referred to as the compounds of the invention.

Preferred compounds of the invention are the compounds of formula (I) and pharmaceutically acceptable salts and solvates thereof.

Preferably, R⁰ is C₁-C₆ alkylene.
Preferably, R⁰ is C₁-C₃ alkylene.
Preferably, R⁰ is ethylene.

Preferably,-R¹ is phenyl substituted by -SO_{y}R⁵, (C₁-C₆ alkylene)-SO_{y}R⁵, -SO_{y}CF₃, -(C₁-G₆ alkylene)-SO_{y}CF₃, -CO₂R⁵, -(C₀-C₆ alkylene)-CO₂R⁵ or OCF₃, said phenyl being optionally additionally substituted by halo, -CN, -COR⁵, -CONR⁵R⁵, -SO₂NR⁶R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.
Preferably, R¹ is phenyl substituted by -SO_{y}R⁵, (C₁-C₆ alkylene)-SO_{y}R⁵, -CO₂R⁵, or -(C₀-C₆ alkylene)-CO₂R⁵, said phenyl being optionally additionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.
Preferably, R¹ is phenyl substituted by -SO_{y}R⁵, (C₁-C₆ alkylene)-SO_{y}R⁵, -CO₂R⁵, or -C₀-C₆ alkylene)-CO₂R⁵.
Preferably, R¹ is phenyl substituted by -SO_{y}(C₁-C₂ alkyl) or -(C₁-C₂ alkylene)-SO_{y}(C₁-C₂ alkyl).

Preferably, R² is H, C₁-C₆ alkyl, C₃-C₆ alkenyl, phenyl, benzyl or R⁹, said phenyl, benzyl or C₁-C₆ alkyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, R⁸ or R⁹.
Preferably, R² is H C₁-C₆ alkyl, phenyl or benzyl, said C₁-C₆ alkyl being optionally substituted by halo, -OR⁵, -OR¹⁰ or -NH₂.
Preferably, R² is H or C₁-C₃ alkyl optionally substituted by -OH or -NH₂.
Preferably, R² is H.

Preferably, R³ is H, C₁-C₆ alkyl or C₃-C₇ cycloalkyl, said C₁-C₆ alkyl being optionally substituted by halo, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ or R⁹.
Preferably, R³ is H, C₁-C₆ alkyl or C₃-C₇ cycloalkyl.
Preferably, R³ is H, C₁-C₃ alkyl or cyclopropyl.
Preferably, R³ is methyl, ethyl or cyclopropyl.

Preferably, R⁴ is phenyl optionally substituted by R⁸, halo, -CN, C₁-C₆ alkyl, C₁-C₆ haloalkyl, C₃-C₇ cycloalkyl or C₁-C₆ alkoxy.
Preferably, R⁴ is phenyl substituted by R⁸, halo, -CN, C₁-C₆ alkyl, or C₁-C₆ alkoxy.
Preferably, R⁴ is phenyl substituted by halo, -CN, C₁-C₆ alkyl, or C₁-C₆ alkoxy.
Preferably, R⁴ is phenyl substituted by -CN.
Preferably, R⁴ is 3,5-dicyanophenyl.

Preferably, R⁵ is H or C₁-C₄ alkyl.
Preferably, R⁵ is C₁-C₂ alkyl.

Preferably, R⁸ is pyrrolyl, imidazolyl, pyrazolyl, 1,2,3-triazolyl, 1,2,4-triazolyl, tetrazolyl, oxazolyl, isoxazolyl, thiazolyl, isothiazolyl, 1,2,4-oxadiazolyl, 1,3,4-oxadiazolyl, furanyl, thienyl, pyridinyl, pyridazinyl, pyrimidinyl or pyrazinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OR⁵, -NR⁵R⁵ or C₁-C₆ alkyl.
Preferably, R⁸ is imidazolyl, pyrazolyl, 1,2,4-triazolyl, 1,2,4-oxadiazolyl, 1,3,4-oxadiazolyl, pyridinyl, pyrazinyl or pyrimidinyl, each being optionally substituted by -OH, -NH₂ or methyl.

Preferably, R⁹ is azetidinyl, tetrahydropyrrolyl, piperidinyl, azepinyl, oxetanyl, tetrahydrofuranyl, tetrahydropyranyl, oxepinyl, morpholinyl, piperazinyl or diazepinyl, each being optionally substituted by oxo, C₁-C₆ alkyl, C₃-C₇ cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.
Preferably, R⁹ is azetidinyl, piperidinyl, tetrahydrofuranyl, piperazinyl or morpholinyl, each being optionally substituted by oxo, C₁-C₆ alkyl, C₃-C₇ cycloalkyl, -SO₂R⁵, -CONR⁵R⁵, -COOR₅, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN.
Preferably, R⁹ is azetidinyl, piperidinyl, tetrahydrofuranyl, piperazinyl or morpholinyl, each being optionally substituted by C₁-C₆ alkyl, -SO₂R⁵, -CONR⁵R⁵, -COOR⁶, -CO-(C₁-C₆ alkylene)-QR⁵ or -COR⁵ and optionally substituted on a carbon atom which is not adjacent to a heteroatom by -OR⁶ or -NR⁵COR⁵.
Preferably, R⁹ is azetidinyl, piperidinyl, tetrahydrofuranyl, piperazinyl or morphoninyl, each being optionally substituted by -CH₃, -SO₂CH₃, -CONH₂, -COOCH₃, -COCH₂OCH₃ or -COCH₃ and optionally substituted on a carbon atom which is not adjacent to a heteroatom by -OCH₃ or -NHCOCH₃.

Preferably, R¹⁰ is C₁-C₄ alkyl substituted by R⁸, R⁹, -OR⁵, -CONR⁵R⁵, -NR⁵COR⁵ or -NR⁵R⁵.
Preferably, R¹⁰ is C₁-C₄ alkyl substituted by R⁹, -OR⁵, -NR⁵COR⁵ or -NR⁵R⁵.
Preferably, R¹⁰ is C₁-C₂ alkyl substituted by tetrahydrofuranyl, -OCH₃, -NHCOCH₃ or -NH₂.

Preferably, R¹¹ is phenyl substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl.
Preferably, R¹¹ is phenyl substituted by halo, -CN, -CONR⁵R⁵, -SO₂NR⁵R⁵ or -OR⁵.
Preferably, R¹¹ is phenyl substituted by -OR⁵.
Preferably, R¹¹ is phenyl substituted by C₁-C₂ alkoxy.

Preferred compounds according to the invention include all combinations of the preferred definitions for individual substituents given above.

Preferred compounds according to the invention include those of formula (Ia), 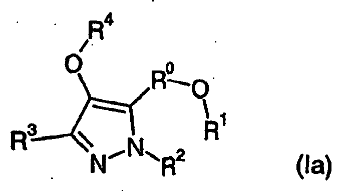 and pharmaceutically acceptable salts or solvates thereof, wherein:
R⁰ is C₁-C₃ alkylene;
R¹ is phenyl substituted by -SO_{y}R⁵, (C₁-C₆ alkylene)-SO_{y}R⁵, -CO₂R⁵, or -(C₀-C₆ alkylene)-CO₂R⁵, said phenyl being optionally additionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl;
R² is H or C₁-C₃ alkyl optionally substituted by -OH or -NH₂;
R³ is H, C₁-C₃ alkyl or cyclopropyl;
R⁴ is phenyl substituted by halo, -CN, C₁-C₆ alkyl, or C₁-C₆ alkoxy;
R⁵ is H or C₁-C₄ alkyl; and
y is 0, 1 or 2.
   Within the compounds of formula (la), preferably R¹ is phenyl substituted by -SO_{y}R⁵, (C₁-C₆ alkylene)-SO_{y}R⁵, -CO₂R⁵, or -C₀-C₆ alkylene)-CO₂R⁵
Preferred compounds of the invention are:
   5-[(3-Ethyl-5-{2-[4-(methylsulfanyl)phenoxy]ethyl}-1*H*-pyrazol-4-yl)oxy]isophthalonitrile;
   5-[(3-Ethyl-5-{2-[4-(methylsulfonyl)phenoxy]ethyl}-1*H*-pyrazol-4-yl)oxy]isophthalonitrile;
   and pharmaceutically acceptable salts, solvates or derivatives thereof.
   The compounds of the invention may have advantages over those of the prior art with regard to a number of useful properties or combination thereof, such as potency, duration of action, pharmacokinetics, spectrum of activity, side effect profile, solubility, chemical stability, and so on.
   The compounds of the invention may be prepared by any method known in the art for the preparation of compounds of analogous structure. The compounds of the invention can be prepared by the procedures described in the methods below, or by the specific methods described in the Examples, or by similar methods to either. The invention also encompasses any one or more of these processes for preparing the compounds of the invention, in addition to any novel intermediates used therein.
   In the following methods R⁰ to R⁴ are as previously defined for a compound of formula (I), unless otherwise stated, and Lg, Lg¹ and Lg² are leaving groups, such as halogen (e.g. Cl) or sulfonate ester (e.g. trifluoromethanesulfonate or methanesulfonate).
   Compounds of formula (I) may be prepared according to Scheme 1. 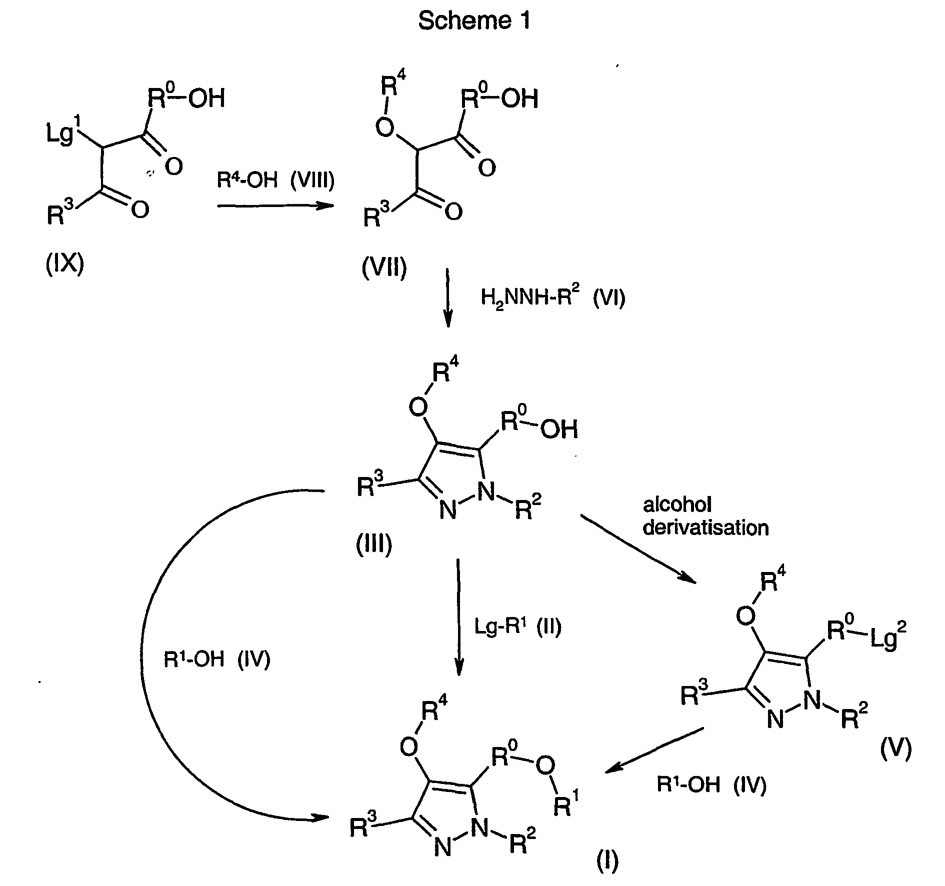
   According to Scheme 1, compounds of formula (I) may be prepared by the reaction of a compound of formula (V) with an alcohol of formula (IV) under conventional conditions. Conveniently, the reaction is effected in the presence of a base, such as a trialkylamine (e.g. triethylamine), a carbonate (e.g. potassium or caesium carbonate) or hydroxide (e.g. sodium hydroxide); a solvent, such as a haloalkane (e.g. dichloromethane); and at ambient to elevated temperature, such as under reflux.
   Compounds of formula (V) may be prepared from compounds of formula (III) by derivatising the hydroxy group therein to provide a leaving group (Lg²). Conveniently, Lg² is a reactive ester group, such as a sulphonic ester group, (e.g. methanesulphonate). Conveniently, the reaction is effected in the presence of a derivatising agent, such as an alkylsulphonyl halide, (e.g. methanesulphonyl chloride); a base, such as a trialkylamine base (e.g. triethylamine); a solvent such, such as a halogenated alkane (e.g. dichloromethane); and at ambient to elevated temperature, such as ambient temperature.
   Compounds of formula (III) may be prepared by the reaction of a compound of formula (VII) with a hydrazine of formula (VI), or a salt or hydrate thereof. Conveniently, the reaction is effected in a solvent, such as a protic solvent (e.g. acetic acid); at ambient to elevated temperature, such as ambient temperature; and optionally in the presence of an acid (e.g. acetic acid) or a base, such as a tertiary amine (e.g. triethylamine).
   Compounds of formula (VII) may be prepared by the reaction of a compound of formula (IX) with an alcohol of formula (VIII). Conveniently, the reaction is effected in the presence of a solvent, such as a polar solvent (e.g. acetone); a base, such as an inorganic base, preferably a metal carbonate (e.g. potassium or caesium carbonate); optionally, a nucleophilic catalyst, such as sodium iodide or tetrabutylammonium iodide; and at ambient to elevated temperature, such as elevated temperature (e.g. under reflux).
   Ketoesters of formula (IX) are either commercially available, known in the literature, or may be prepared by conventional methods (e.g., where Lg¹ is Cl, by the chlorination of corresponding ketoesters, for instance using sulphonyl chloride).
   According to Scheme 1, compounds of formula (I) may also be prepared by the reaction of an alcohol of formula (III) with a compound of formula (II) under conventional conditions. Conveniently, the reaction is effected in the presence of a base, such as an inorganic base, preferably a metal carbonate (e.g. potassium carbonate); optionally a solvent, such as a polar aprotic solvent (e.g. N,N-dimethylacetamide); optionally a catalyst, such as a copper(I) catalyst; and at ambient to elevated temperature, such as elevated temperature (e.g. under reflux).
   According to Scheme 1, compounds of formula (I) may also be prepared by the reaction of an alcohol of formula (III) with an alcohol of formula (IV) under dehydrating conditions, such as afforded by the Mitsunobu reaction. Conveniently, the reaction is effected in the presence of an azodicarboxylate, such as diisopropylazodicarboxylate; triphenylphosphine; a solvent, such as an ether (e.g. tetrahydrofuran); and at reduced to ambient temperature, such 0°C to ambient temperature.
   Compounds of formula (I) in which R³ is halo can be prepared from a compound of formula (X) 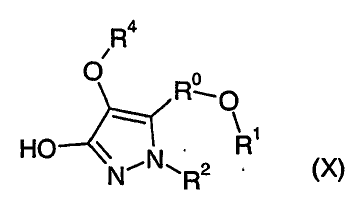 under conventional conditions. Conveniently, the reaction is effected by an inorganic acid halide, such as an inorganic acid chloride (e.g. POCl₃); optionally in the presence of a solvent, such as a polar aprotic solvent (e.g. N,N-dimethylformamide); and at reduced to ambient temperature, such as ambient temperature.
   Compounds of formula (X) may be prepared using the routes described above, *mutatis mutandis.*
   It will be appreciated by those skilled in the art that, in many cases, it may be necessary or desirable to protect one or more sensitive functional groups, for example hydroxy groups, in the preparation of compounds of formula (I), as for example set out in Scheme 1. In particular, it may be necessary or desirable to protect the -R⁰-OH group of compounds of formulae (IX), (VII) and (III), and to deprotect the group prior to further transformation of a compound of formula (III). Likewise, when R² is H, it may be necessary or desirable to protect the pyrazole NH group of compounds of formulae (III), (V) and (I) and to deprotect the group to provide a compound of formula (I). Examples of suitable protecting groups will be apparent to the skilled person. See, for instance, 'Protecting groups in Organic Synthesis (Second Edition)' by Theodora W. Green and Peter G. M. Wuts, 1991, John Wiley and Sons (in particular pages 10 - 118, relating to protection for the hydroxyl group, and pages 309 to 405, relating to protection for the amino group, both of which describe methods for protection and subsequent deprotection), incorporated herein by reference.
   It will be appreciated by those skilled in the art that compounds of formula (I) containing an -OH, -NH- or -NH₂ group may be prepared by deprotection of the corresponding compound bearing an -OP¹, -NP¹- or -NHP¹ group, respectively, wherein the group P¹ is a suitable protecting group. Such compounds bearing an -OP¹, -NP¹- or -NHP¹ group may be prepared using the routes described above, *mutatis mutandis.*

It will be appreciated by those skilled in the art that, in many cases, compounds of formula (I) may be converted into other compounds of formula (I) by functional group transformations, including for example the following interconversions.

Compounds of formula (I) in which R¹ is phenyl substituted by C₁-C₆ alkylsulfinyl or C₁-C₆ alkylsulfonyl may be prepared by oxidation of the corresponding compound of formula (I) wherein R¹ is phenyl substituted by C₁-C₆ alkylsulfanyl. The skilled artisan will appreciate that, depending on the severity of the oxidatitive conditions employed, such alkylsulfinyls of formula (I) may be partially oxidised to corresponding alkylsulfinyls, or fully oxidised to corresponding alkylsulfonyls. For the preparation of C₁-C₆ alkylsulfinyl compounds the oxidation is, conveniently, effected in the presence of an oxidising agent, such as oxone; optionally in the presence of a moderator, such as wet alumina; a solvent, such as a haloalkane (e.g. dichloromethane); and carried out at ambient to elevated temperature, such as under reflux. For the preparation of C₁-C₆ alkylsulfonyl compounds the oxidation is, conveniently, effected in the presence of an oxidising agent, such as oxone; a solvent, such as an aqueous alcohol (e.g. aqueous methanol); and at reduced to ambient temperature (e.g. 0°C).

Compounds of formula (I) in which R² is optionally substituted C₁-C₆ alkyl may be prepared from compounds of formula (I) in which R² is H by reaction with an alkylating agent. Suitable alkylating agents include bromoacetonitrile, ethyl 4-chloroacetoacetate, methyl bromoacetate and chloroethylamine hydrochloride. Conveniently, alkylation is effected in the presence of a suitable solvent, such as an alcohol (e.g. ethanol) or a polar aprotic solvent (e.g. N,N-dimethylformamide); a base, such as a metal hydride (e.g. sodium hydride) or metal alkoxide (e.g. sodium ethoxide); and at ambient to elevated temperature, such as under reflux.

Compounds of formula (I) in which R² or R³ contains a hydroxy group may be prepared from the corresponding compound of formula (I) in which R² or R³ contains an ester group by reduction. Conveniently, the reduction is effected by a metal hydride, such as lithium aluminium hydride; in a solvent, such as an ether (e.g. diethyl ether); and at reduced temperature, such as from -78°C to 0°C.

Compounds of formula (I) in which R² or R³ are substituted by a heterocycle of formula R⁸ and R⁹ may be prepared by standard heterocycle-forming reactions well known to the skilled man (see, for example, Advanced Organic Chemistry, 3rd Edition, by Gerry March or Comprehensive Heterocyclic Chemistry, A.R. Katritzky, C.W. Rees, E.F.V. Scriven, Volumes 1-11).

Compounds of formula (I) in which R³ is -CO₂H may be prepared by hydrolysis of a corresponding compound of formula (I) in which R³ is -CO₂R⁵. Conveniently, the reaction is effected in the presence of a solvent, such as an alcohol (e.g. aqueous ethanol), or an ether (e.g. aqueous 1,4-dioxan); and in the presence of a base, such as a metal hydroxide (e.g. sodium hydroxide). The skilled artisan will appreciate that such an acid may be converted into a primary amide by reaction with ammonia and a suitable coupling agent, such as a carbodiimide, e.g. dicyclohexylcarbodiimide, and that such a primary amide may then be converted into a nitrile by dehydration with a suitable dehydrating agent, such as phosphoryl chloride.

Compounds of formula (I) in which R³ is C₁-C₆ alkyl may be converted into the compounds of formula (I) in which R³ is C₁-C₆ alkyl substituted by halo (such as bromo), by halogenation, using a suitable halogenating agent. Conveniently the reaction is effected in the presence of a solvent, such as a haloalkane (e.g. dichloromethane) and at ambient temperature. Suitable halogenating agents include halogens (e.g. bromine) or N-halosuccinimides (e.g. N-bromsuccinimide).

Compounds of formulae (II), (IV) and (VI) and (VIII) are either commercially available, known in the literature or easily prepared by methods well known to those skilled in the art, such as those described in the Preparations hereinafter.

Compounds of formulae (III), (V) or (X) are key intermediates. Compounds of formulae (V) and (X) form a further a further aspect of the invention.

The compounds of the invention can be administered alone, but will generally be administered in admixture with a suitable pharmaceutical excipient, diluent or carrier selected with regard to the intended route of administration and standard pharmaceutical practice.

For example, the compounds of the invention can be administered orally, buccally or sublingually in the form of tablets, capsules, multi-pardcuiates, gets, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations of the compounds of the invention may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain from 0.01 mg to 500mg of active compound whilst tablet fill weights may range from 50mg to 1000mg. An example of a formulation for a 10mg tablet is illustrated below:

| Ingredient | %w/w |
|---|---|
| Compound of the invention | 10.000* |
| Lactose | 64.125 |
| Starch | 21.375 |
| Croscarmellose sodium | 3.000 |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds of the invention can also be administered parenterally, for example, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the invention will usually be from 0.01 to 30 mg/kg, preferably from 0.01 to 5 mg/kg (in single or divided doses).

Thus tablets or capsules of the compound of the invention may contain from 1 to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that, in the treatment of certain conditions the compounds of the invention may be taken as a single dose as needed or desired.

The compounds of invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Alternatively, the compounds of the invention can be administered in the form of a suppository or pessary, or they may be applied topically in the form of a gel, hydrogel, lotion, solution, cream, ointment or dusting powder. The compounds of the invention may also be dermally or transdermally administered, for example, by the use of a skin patch. They may also be administered by the pulmonary or rectal routes.

They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO91/11172, WO94/02518 and WO98/55148.

It is to be appreciated that all references herein to treatment include curative, palliative and prophylactic treatment.

Oral administration is preferred.

Included within the scope of the invention are compositions containing a compound of the invention along with one or more additional therapeutic agents. Such a combination therapy is especially useful for the prevention and/or treatment of infection by HIV and related retroviruses which may evolve rapidly into strains resistant to any monotherapy. Alternatively, additional therapeutic agents may be desirable to treat diseases and conditions which result from or accompany the disease being treated with the compound of the invention. For example, in the treatment of an HIV or related retroviral infection, it may be desirable to additionally treat opportunistic infections, neoplasms and other conditions which occur as a result of the immuno-compromised state of the patient being treated.

Preferred combinations of the invention include simultaneous or sequential treatment with a compound of the invention and one or more:
(a) reverse transcriptase inhibitors such as abacavir, adefovir, didanosine, lamivudine, stavudine, zalcitabine and zidovudine;
(b) non-nucleoside reverse transcriptase inhibitors such as capavirine, delavirdine, efavirenz, and nevirapine;
(c) HIV protease inhibitors such as indinivir, nelfinavir, ritonavir, and saquinavir;
(d) CCR5 antagonists such as TAK-779 or UK-427,857;
(e) CXCR4 antagonists such as AMD-31 00;
(f) integrase inhibitors, such as L-870,810 or S-1360;
(g) inhibitors of viral fusion such as T-20;
(h) investigational drugs such as trizivir, KNI-272, amprenavir, GW-33908, FTC, PMPA, MKC-442, MSC-204, MSH-372, DMP450, PNU-140690, ABT-378, KNI-764, DPC-083, TMC-120 or TMC-125;
(i) antifungal agents, such as fluconazole, itraconazole or voriconazole; or
(j) antibacterial agents, such as azithromycin.

The activity of the compounds of the invention as reverse transcriptase inhibitors may be measured using the following assay.

### Inhibition of HIV-1 reverse transcriptase enzyme

Using purified recombinant HIV-1 reverse transcriptase (RT, EC, 2.7.7.49) obtained by expression in Escherichia Coli, a 96-well plate assay system is established for assaying a large number of samples using either the Poly(rA)-oligo(dT) Reverse Transcriptase [3H]-SPA enzyme assay system (Amersham NK9020) or the [3H]-flashplate enzyme assay system (NEN - SMP 103) and following the manufacturer's recommendations. The compounds are dissolved in 100% DMSO and diluted with the appropriate buffer to a 5% final DMSO concentration. The inhibitory activity is expressed in percent inhibition relative to DMSO control. The concentration at which compound inhibits reverse transcriptase by 50% is expressed as the IC₅₀ of the compound.

The compound of Examples 1 and 3, when tested according to the above procedure, had an IC₅₀ values of, respectively, 2 and 45 nanomolar.

Thus the invention provides:
(i) a compound of formula (I) or a pharmaceutically acceptable salt or solvate thereof;
(ii) a process for the preparation of a compound of formula (I) or a pharmaceutically acceptable salt or solvate thereof;
(iii) a pharmaceutical composition including a compound of formula (I) or a pharmaceutically acceptable salt or solvate thereof, together with a pharmaceutically acceptable excipient, diluent or carrier;
(iv) a compound of formula (I) or a pharmaceutically acceptable salt, solvate or composition thereof, for use as a medicament;
(v) a compound of formula (I) or a pharmaceutically acceptable salt, solvate or composition thereof, for use as a reverse transcriptase inhibitor or modulator;
(vi) a compound of formula (I) or a pharmaceutically acceptable salt, solvate or composition thereof, for use In the treatment of an HIV or genetically-related retroviral infection, or a resulting acquired immune deficiency syndrome (AIDS);
(vii) a use of the compound of formula (I) or of a pharmaceutically acceptable salt, solvate or composition thereof, for the manufacture of a medicament having reverse transcriptase inhibitory or modulating activity;
(viii) the use of a compound of formula (I) or of a pharmaceutically acceptable salt, solvate or composition thereof, for the manufacture of a medicament for the treatment of an HIV or genefically-related retroviral infection, or a resulting acquired immune deficiency syndrome (AIDS);
(ix) certain novel intermediates disclosed herein.

The following Examples illustrate the preparation of the compounds of formula (I). The synthesis of certain intermediates used therein are described in the Preparations section that follows the Examples.

¹H Nuclear magnetic resonance (NMR) spectra were in all cases consistent with the proposed structures. Characteristic chemical shifts (δ) are given in parts-per-million downfield from tetramethylsilane using conventional abbreviations for designation of major peaks: e.g. s, singlet; d, doublet; t, triplet; q, quartet; m, multiplet; br, broad. The following abbreviations have been used: HRMS, high resolution mass spectrometry; hplc, high performance liquid chromatography; nOe, nuclear Overhauser effect; m.p., melting point; CDCl₃, deuterochloroform; D₆-DMSO, deuterodimethylsulphoxide; CD₃OD, deuteromethanol. Where thin layer chromatography (TLC) has been used it refers to silica gel TLC using silica gel 60 F₂₅₄ plates, R_{f} is the distance travelled by a compound divided by the distance travelled by the solvent front on a TLC plate.

### EXAMPLE 1

### 5-[(3-Ethyl-5-{2-[4-(methylsulfanyl)phenoxy]ethyl}-1H-pyrazol-4-yl)oxy]isophthalonitrile

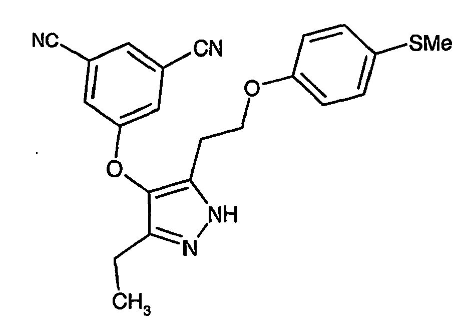

To a stirred solution of the alcohol from Preparation 10 (268mg, 0.95mmol) in tetrahydrofuran (8ml) at 0°C was added 4-(methylmercatpto)phenol (200mg, 1.43mmol), triphenylphosphine (374mg, 1.43mmol) and diisopropylazodicarboxylate (275µl, 1.43mmol). The reaction was allowed to warm to room temperature and was stirred for 18 hours. The mixture was concentrated under reduced pressure and purified by flash chromatography on silica gel eluting with dichloromethane:methanol (95:5, by volume). Column chromatography was repeated eluting with ethyl acetate and then repeated eluting with ethyl acetate:toluene (25:75, by volume) and finally repeated eluting with ethyl acetate:toluene (15:85, by volume) to give the title compound as a colourless oil (153mg).

¹H NMR (400MHz, CDCl₃): δ = 1.20 (t, 3H), 2.44 (s, 3H), 2.50 (q, 2H), 2.97 (t, 2H), 4.15 (t, 2H), 6.71 (d, 2H), 7.20 (d, 2H), 7.38 (s, 2H), 7.53 (s, 1H).
LRMS (atmospheric chemical ionisation): m/z [MH⁺] 405.

### EXAMPLE 2

### 5-[(3-Ethyl-5-{2-[4-(methylsulfinyl)phenoxy]ethyl}-1H-pyrazol-4-yl)oxy]isophthalonitrile

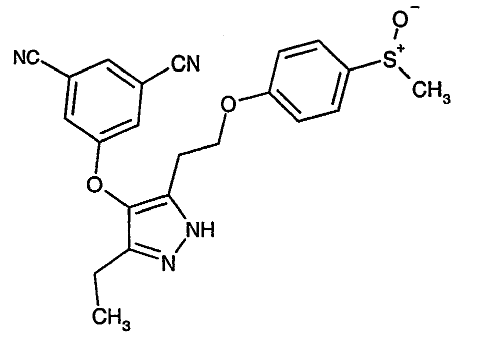

Wet alumina was prepared by adding water (1 ml) to Brockman grade I alumina (5g). A sample of this material (160mg) was added to a stirred solution of the sulfide from Example 1 (106mg, 0.16mmol) in dichloromethane (1 ml), followed by oxone (98mg, 0.16mmol). The reaction mixture was heated at reflux for 45 minutes, before being filtered. The solids were washed with dichloromethane (10ml) and the combined organics were concentrated under reduced pressure. The crude product mixture was purified by flash chromatography on silica gel eluting with dichloromethane:methanol (100:0, 98:2 and 96:4, by volume) to give the title compound as a colourless oil (25mg).

¹H NMR (400MHz, CDCl₃): δ = 1.19 (t, 3H), 2.50 (q, 2H), 2.71 (s, 3H), 2.99 (t, 2H), 4.23 (t, 2H), 6.92 (d, 2H), 7.39 (s, 2H), 7.55 (s, 1H), 7.56 (d, 2H).
LRMS (atmospheric pressure chemical ionisation): m/z [MH⁺] 421
Accurate Mass: Found: 421.1323 [MH⁺]; C₂₂H₂₁N₄O₃S requires 421.1329 [MH⁺].

### EXAMPLE 3

### 5-[(3-Ethyl-5-{2-[4-(methylsulfonyl)phenoxy]ethyl}-1H-pyrazol-4-yl)oxy]isophthalonitrile

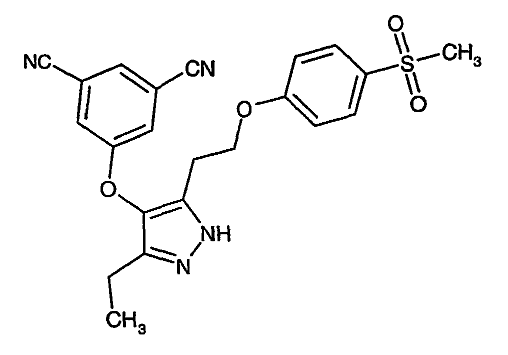

To a stirred solution of the sulfide from Example 1 (90mg, 0.22mmol) in methanol (2ml) at 0°C was added a solution of oxone (205mg, 0.33mmol) in water (2ml). The viscous suspension was further diluted with methanol (2ml). After 4 hours the reaction mixture was concentrated under reduced pressure and the residue was partitioned between dichloromethane (20ml) and water (20ml). The organic layer was dried over magnesium sulfate, filtered and concentrated under reduced pressure. The crude product mixture was purified by flash chromatography on silica gel eluting with pentane:ethyl acetate (50:50, by volume) to give the title compound (70mg) as a white powder, m.p. 88-90°C.

¹H NMR (400MHz, CDCl₃): δ = 1.20 (t, 3H), 2.51 (q, 2H), 3.00 (t, 2H), 3.03 (s, 3H), 4.27 (t, 2H), 6.92 (d, 2H), 7.39 (s, 2H), 7.56 (s, 1H), 7.84 (d, 2H).
LRMS (atmospheric pressure chemical ionisation): m/z [MH⁺] 437
Microanalysis: Found: C, 60.17; H, 4.60; N, 12.73. C₂₂H₂₀N₄O₄S requires C, 60.54; H, 4.62; N, 12.84%.

### EXAMPLE 4

### 5-[(3-Ethyl-5-{2-[3-(methylsulfanyl)phenoxy]ethyl}-1H-pyrazol-4-yl)oxy]isophthalonitrile

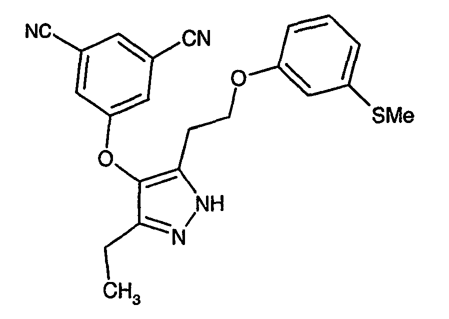

To a stirred solution of the alcohol from Preparation 10 (395mg, 1.40mmol) in tetrahydrofuran (14ml) at 0°C was added 3-(methylmercatpto)phenol (196mg, 1.40mmol), triphenylphosphine (367mg, 1.40mmol) and diethylazodicarboxylate (276µl, 1.60mmol). The reaction was allowed to warm to room temperature and was stirred for 18 hours. The mixture was concentrated under reduced pressure and purified by flash chromatography on silica gel eluting with pentane:ethyl acetate (9:1, 5:1 and 3:1 by volume). Column chromatography was repeated eluting with dichloromethane:acetonitrile (100:0, 99:1, 98:2 and 97:3 by volume) to give the title compound as a colourless oil (207mg, approximately 60% pure w/w) which was contaminated with triphenylphosphine oxide and the reduced diethylazodicarboxylate.

¹H NMR (400MHz, CDCl₃): δ = 1.16 (t, 3H), 2.45 (s, 3H), 2.48 (q, 2H), 2.96 (t, 2H), 4.16 (t, 2H), 6.53 (d, 1H), 6.62 (s, 1H), 6.81 (s, 1H), 7.13 (t, 1H), 7.35 (s, 2H). 7.53 (s, 1H).
LRMS (atmospheric chemical ionisation): m/z [MH⁺] 405.

### EXAMPLE 5

### 5-[(3-Ethyl-5-{2-[3-(methylsulfinyl)phenoxy]ethyl}-1H-pyrazol-4-yl)oxy]isophthalonitrile

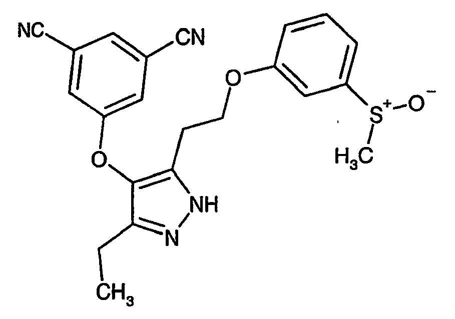

Wet alumina was prepared by adding water (1 ml) to Brockman grade I alumina (5g). A sample of this material (320mg) was added to a stirred solution of the sulfide from Example 4 (217mg, 0.32mmol) in dichloromethane (2ml), followed by oxone (196mg, 0.32mmol). The reaction mixture was heated at reflux for 45 minutes, before being filtered. The solids were washed with dichloromethane (10ml) and the combined organics were concentrated under reduced pressure. The crude product mixture was purified by flash chromatography on silica gel eluting with dichloromethane:methanol (100:0 , 98:2 and 96:4, by volume) to give the title compound as a colourless oil (69mg).

¹H NMR (400MHz, CDCl₃): δ = 1.19 (t, 3H), 2.12 (q, 2H), 2.73 (s, 3H), 2.99 (t, 2H), 4.27 (t, 2H), 6.88 (d, 1H), 7.10 (d, 1H), 7.20 (m, 1H), 7.37 (t, 1H), 7.39 (s, 2H), 7.54 (s, 1H).
LRMS (atmospheric pressure chemical ionisation): m/z [MH⁺] 421
Accurate Mass: Found: 421.1330 [MH⁺]; C₂₂H₂₁N₄O₃S requires 421.1329 [MH⁺].

### EXAMPLE 6

### 5-[(3-Ethyl-5-{2-[3-(methylsulfonyl)phenoxy]ethyl}-1H-pyrazol-4-yl)oxy]isophthalonitrile

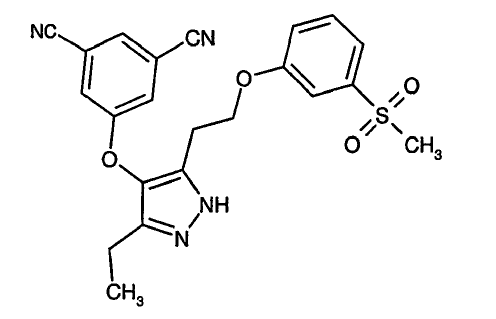

To a stirred solution of the sulfoxide from Example 5 (34mg, 0.08mmol) in methanol (1ml) was added a solution of oxone (49mg, 0.08mmol) in water (1 ml). After 2 hours the reaction mixture was concentrated under reduced pressure and the residue was partitioned between dichloromethane (5ml) and water (5ml). The organic layer was dried over magnesium sulfate, filtered and concentrated under reduced pressure. The crude product mixture was purified by flash chromatography on silica gel eluting with dichloromethane:methanol (100:0, 99:1 then 98:2, by volume) to give the title compound as a colourless oil (12mg).

¹H NMR (400MHz, CDCl₃): δ = 1.21 (t, 3H), 2.52 (q, 2H), 3.01 (t, 2H), 3.06 (s, 3H), 4.27 (t, 2H), 7.08 (d, 1H), 7.34 (t, 1H), 7.41 (s, 2H), 7.46 (t, 1H), 7.53 (m, 1H), 7.57 (s, 1H).
LRMS (atmospheric pressure chemical ionisation): m/z [MH⁺] 437
Accurate Mass: Found: 437.1279 [MH⁺]; C₂₂H₂₁N₄O₃S requires 421.1278 [MH⁺].

### PREPARATION 1

### 1.3-Dibromo-5-methoxybenzene

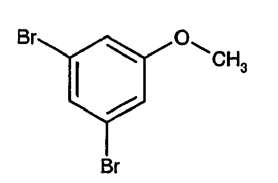

Sodium methoxide (8.80ml of a 4.5M solution in methanol, 39.6mmol) was added dropwise to a stirred solution of 3,5-dibromofluorobenzene (5.00g, 19.0mmol) in *N*,*N*-dimethylformamide (95ml) at 0°C under nitrogen. The reaction was allowed to warm to room temperature, stirred for 1 hour and then concentrated under reduced pressure. The residue was dissolved in ether (500ml) and the resulting solution was washed with water (3x300ml) and brine (300ml), dried over magnesium sulphate, filtered and concentrated under reduced pressure to provide the title compound (5.13g) as a white solid.
¹H-NMR (300MHz, CDCl₃): δ = 3.79 (s, 3H), 7.00 (s, 2H), 7.26 (s, 1H).
LRMS (thermospray): m/z [MH⁺] 266.
Microanalysis: Found: C, 31.56; H, 2.29. C₇H₆OBr₂ requires C, 31.62; H, 2.27%.

### PREPARATION 2

### 3.5-Dicyanomethoxybenzene

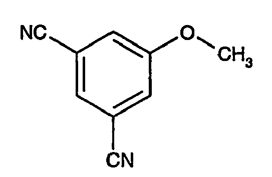

Tris(dibenzylideneacetone)dipalladium(0) (6.53g, 7.15mmol) was added in one portion to a stirred solution of the bromide of Preparation 1 (38.0g, 143mmol), 1,1'-bis(diphenylphosphino)ferrocene (9.3g, 16.8mmol) and zinc cyanide (20.0g, 172mmol) in *N,N*-dimethylformamide (300ml) at room temperature under nitrogen. The reaction was heated at 100°C for 14 hours and cooled to room temperature. Water (1500ml) was added and the mixture was extracted with ethyl acetate (3x500ml). The combined organics were filtered and the filtrate was washed with water (500ml), dried over magnesium sulphate, filtered and concentrated under reduced pressure. The resulting solid was triturated with toluene (1000ml) to provide the title compound (18.0g) as a tan solid.
¹H-NMR (300MHz, CDCl₃): δ = 3.83 (3H, s), 7.31 (2H, s), 7.48 (1H, s).

### PREPARATION 3

### 3,5-Dicyanohdroxybenzene

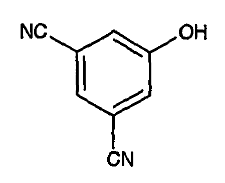

The nitrile of Preparation 2 (9.60g, 60.7mmol) was added portionwise to a stirred suspension of aluminium trichloride (32.4g, 243mmol) in dichloromethane (250ml) at 0°C under nitrogen. The suspension was heated to 45°C and stirred for 6 days. The reaction was cooled to room temperature and cautiously poured onto ice (450ml). Concentrated hydrochloric acid (450ml) was added dropwise and the resulting suspension was stirred for 10 minutes at room temperature. The resulting solid was collected by filtration, washed with water and dried over phosphorus pentoxide to provide the title compound (7.83g) as a tan solid containing approximately 10 % starting material by ¹H-NMR and microanalysis.
¹H-NMR (400MHz, CDCl₃): δ = 7.36 (m, 2H), 7.56 (m, 1H).

### PREPARATION 4

### 3-Oxoeentanoic acid

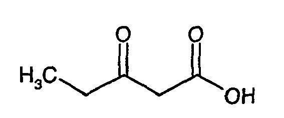

Sodium hydroxide (54g, 1.35mol) was added portionwise to a solution of 3-oxo-pentanoic acid methyl ester (80g, 0.62mol) in tetrahydrofuran (300ml) and water (300ml) at 0°C. The reaction was allowed to warm to room temperature and was stirred for 18 hours. The reaction mixture was washed with diethylether (500ml) and the aqueous phase was acidified to pH1 at 0°C with concentrated hydrochloric acid (140ml). The aqueous phase was extracted with dichloromethane (2x300ml) and the combined organic extracts dried over magnesium sulphate and concentrated under reduced pressure to provide the title compound (44g) as a white solid.
¹H NMR (400MHz, CDCl₃): δ = 1.12 (t, 3H), 2.59 (q, 2H), 3.49 (s, 2H).

### PREPARATION 5

### 3-(Benzyloxy)propanoic acid

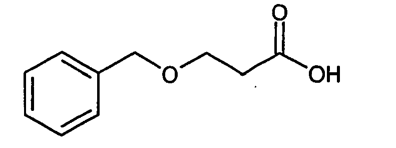

Sodium metal (249mg, 10.8mmol) was added to benzyl alcohol (30g, 278mmol) at room temperature under nitrogen and the reaction was stirred for 30 minutes. Methyl acrylate (25.9ml, 259mmol) was then added dropwise and the reaction was stirred at room temperature for 18h. After quenching with saturated aqueous ammonium chloride solution (200ml) the mixture was extracted with ethyl acetate (2x300ml) and the combined organic extracts were washed with brine (100ml), dried over magnesium sulphate and concentrated under reduced pressure. The residual oil was dissolved in ethanol (300ml) and 1 M aqueous sodium hydroxide solution (300ml) was added dropwise. After 3 hours the ethanol was removed under reduced pressure and the aqueous residue was washed with dichloromethane (200ml). The aqueous phase was then acidified with 2N aqueous hydrochloric acid (150ml), extracted with dichloromethane (2x250ml) and the combined organic extracts were dried over magnesium sulphate and concentrated under reduced pressure. The residual oil was dissolved in 10% aqueous potassium carbonate solution (300ml), washed with diethylether (300ml) and the aqueous phase was acidified to pH1 using concentrated hydrochloric acid. The mixture was then extracted with dichloromethane (2x300ml) and the combined organic extracts were dried over magnesium sulphate and concentrated under reduced pressure to provide the title compound (44.4g) as a colourless oil.
¹H NMR (300MHz, CDCl₃): δ = 2.67 (t, 2H), 3.89 (t, 2H), 4.58 (s, 2H), 7.18 (m, 5H).

### PREPARATION 6

### (4Z)-1-(Benzyloxy)-5-hydroxy-4-hepten-3-one

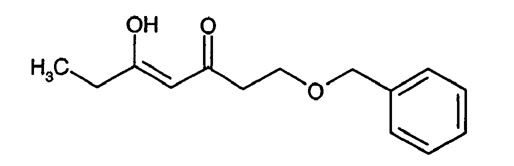

A suspension of magnesium turnings (1.74g, 71.6mmol) in methanol (85ml) was heated at reflux under nitrogen for 1.5 hours, cooled to room temperature and the β-keto acid from Preparation 4 (16.6g, 143mmol) was added. The reaction was stirred for 1.5 hours and the solvent was removed under reduced pressure to give the magnesium salt of the acid as a white solid. Meanwhile, the acid from Preparation 5 (12.9g, 71.6mmol) was dissolved in N,N'-dimethylformamide (150ml) and carbonyldiimidazole (12.8g, 78.8mmol) was added portionwise under nitrogen at room temperature. This was stirred for 1 hour and then the magnesium salt from above was added as a solution in N,N'-dimethylformamide (50ml). Evolution of gas was noted, and the reaction was allowed to stir at room temperature for 18 hours. The mixture was concentrated under reduced pressure and the residual orange oil was dissolved in dichloromethane (300ml), washed with 0.5M aqueous hydrochloric acid (250ml) containing methanol (10ml) and the aqueous phase was separated and extracted with dichloromethane (2x300ml). The combined organic extracts were washed with brine (300ml) containing methanol (20ml), dried over magnesium sulphate, filtered and concentrated under reduced pressure. The residual orange oil was purified by flash chromatography on silica gel eluting with cyclohexane:ethyl acetate (80:20, by volume) to provide the title compound (12.0g) as an orange oil.
¹H NMR (400MHz, CDCl₃): δ = 1.17 (t, 3H), 2.33 (q, 2H), 2.58 (t, 2H), 3.76 (t, 2H), 4.53 (s, 2H), 5.57 (s, 1H), 7.13 (m, 5H).
LRMS (electrospray) : m/z [MNa⁺] 257.
Microanalysis: Found C, 71.77; H, 7.74. C₁₄H₁₈O₃ requires C, 71.76; H, 7.69%.

### PREPARATION 7

### (4E)-1-(Benzyloxy)-4-chloro-5-hydroxy-4-hepten-3-one

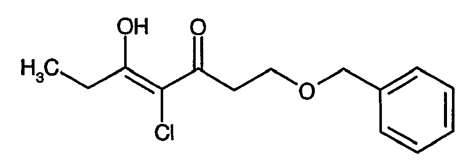

Chlorotrimethylsilane (10ml, 51.3mmol) was added to a solution of the enol from Preparation 6 (4.0g, 17.1mmol) in acetonitrile (25ml) under nitrogen at 0°C. Dimethylsulfoxide (3.6ml, 51.3mmol) followed by tert-butylammonium bromide (275mg, 0.85mmol) were then added and the reaction was stirred at 0°C for 2 hours. The mixture was diluted with water (100ml), extracted with diethylether (100ml) and the organic phase was washed with brine (50ml), dried over magnesium sulphate and concentrated under reduced pressure. The residual pink oil was purified by flash chromatography on silica gel eluting with cyclohexane:ethyl acetate (80:20, by volume) to provide the title compound (3.76g) as a pink oil.
¹H NMR (400MHz, CDCl₃): δ = 1.17 (t, 3H), 2.62 (q, 2H), 2.96 (t, 2H), 3.79 (t, 2H), 4.57 (s, 2H), 7.12 (m, 5H), 15.49 (s, 1H).
LRMS (electrospray) : m/z [MNa⁺] 291.

### PREPARATION 8

### 5-({(1E)-1-[3-(Benzyloxy)propanoyl]-2-hydroxy-1-butenyl}oxy)isophthalonitrile

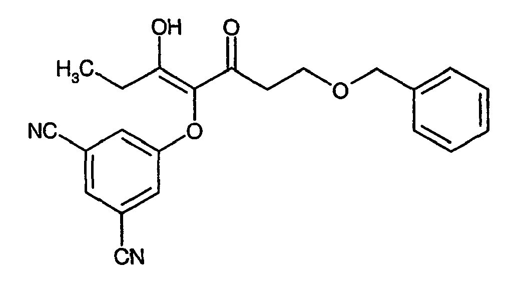

To a stirred solution of the chlorodiketone from Preparation 7 (1.5g, 5.6mmol) in acetone (30ml) was added the phenol from Preparation 3 (0.8g, 5.6mmol) followed by cesium carbonate (1.8g, 5.6mmol). The reaction mixture was heated at reflux for 2 hours and was then allowed to cool to room temperature. The reaction mixture was concentrated under reduced pressure and the residue was diluted with between water (50ml), 2N aqueous hydrochloric acid (50ml) and extracted with dichloromethane (2x100ml). The combined organic components were dried over magnesium sulfate, filtered and concentrated under reduced pressure. The crude product mixture was purified by column chromatography on silica gel eluting with pentane:ethyl acetate (90:10 then 80:20, by volume) to provide the title compound (0.9g) as a yellow oil.

¹H NMR (400MHz, CDCl₃): δ =1.08 (t, 3H), 2.29 (q, 2H), 2.58 (t, 2H), 3.73 (t, 2H), 4.44 (s, 2H), 7.12 (m, 5H), 7.45 (s, 2H), 7.57 (s, 1H), 14.51 (s, 1H).
LRMS (atmospheric pressure chemical ionisation): m/z [M-H⁺] 375.

### PREPARATION 9

### 5-({5-[2-(Benzyloxy)ethyl]-3-ethyl-1H-pyrazol-4-yl}oxy)isophthalonitrile

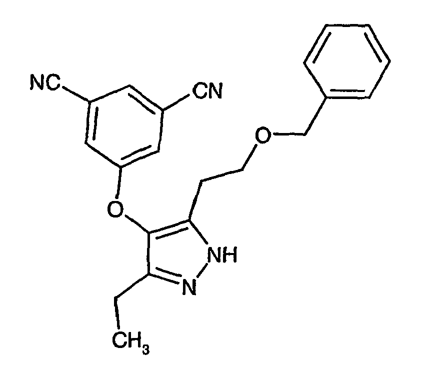

To a stirred solution of the diketone from Preparation 8 (0.85g, 2.3mmol) in acetic acid (20ml) was added hydrazine hydrate (0.17ml, 3.4mmol). After 18 hours the reaction mixture was concentrated under reduced pressure to give a yellow oil (0.85g) which was used without further purification. An analytical sample of the product was purified by preparative HPLC using a Develosil combi-rp C30 50x4.6mm 3µm column eluting with a solvent gradient of 5:95 0.1 % aqueous trifluoroacetic acid in water:acetonitrile to provide the title compound as a colourless oil.
¹H NMR (400MHz, CDCl₃): δ = 1.18 (t, 3H), 2.44 (q, 2H), 2.77 (t, 2H), 3.63 (t, 2H), 4.52 (s, 2H), 7.30 (m, 7H), 7.55 (s, 1H).
LRMS (electrospray) : m/z [MH⁺] 231, [MNa⁺] 253.

### PREPARATION 10

### 5-{[3-Ethyl-5-(2-hydroxyethyl)-1H-pyrazol-4-yl]oxy}isophthalonitrile

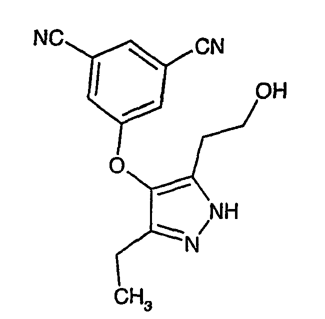

Iron(III)chloride (3.7g, 23mmol) was added to a solution of the crude pyrazole from Preparation 9 (0.85g, 2.3mmol) in dichloromethane (30ml) at room temperature. After stirring for 20 minutes the mixture was diluted with dichloromethane (100ml) and washed with water (50ml). The separated aqueous phase was washed with dichloromethane (100ml) and the combined organic components were dried over magnesium sulphate, filtered and concentrated under reduced pressure. The residue was purified by flash chromatography on silica gel eluting with dichloromethane:methanol (98:2 changing to 95:5, by volume) to provide the title compound (0.50g) as a white solid.

¹H NMR (400MHz, CDCl₃): δ = 1.19 (t, 3H), 2.51 (q, 2H), 2.69 (t, 2H), 3.88 (t, 2H), 7.40 (s, 2H), 7.59 (s, 1H).
LRMS (electrospray): m/z [MH⁺] 283.

## Claims

1. A compound of formula (I) 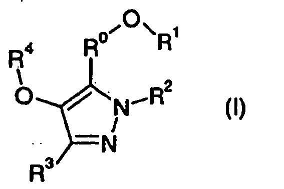 or a pharmaceutically acceptable salt or solvate thereof, wherein:
R⁰ is absent or C₁-C₆ alkylene;
R¹ is phenyl substituted by -SO_{y}R⁵, (C₁-C₆ alkylene)-SO_{y}R⁵. -SO_{y}CF₃, -(C₁-C₆ alkylene)-SO_{y}CF₃, -CO₂R⁵, -(C₀-C₆ alkylene)-CO₂R⁵, OCF₃, a five or six-membered aromatic heterocyclic group containing (i) from 1 to 4 nitrogen heteroatom(s) or (ii) 1 or 2 nitrogen heteroatom(s) and 1 oxygen or 1 sulphur heteroatom (said heterocyclic group being optionally substituted by halo, oxo, -CN, -COR⁵, -CO₂R⁵, -CONR⁵R⁵, -SO_{y}R⁵, -SO_{y}CF₃, -SO₂NR⁵R⁵, -NR⁵SO₂R⁵, -OR⁵, -OCF₃, -NR⁵R⁵, -(C₁-C₆ alkylene)-NR⁵R⁵, C₁-C₆ alkyl, fluoro(C₁-C₆)alkyl or C₃-C₇ cycloalkylor); said phenyl being optionally additionally substituted by halo, -CN, -COR⁵, -CONR⁵R⁵,
-SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl;
R² is H, C₁-C₆ alkyl, C₃-C₆ alkenyl, C₃-C₆ alkynyl, C₃-C₇ cycloalkyl, C₃-C₇ cycloalkenyl, phenyl, benzyl, R⁸ or R⁹, said C₁-C₆ alkyl, C₃-C₇ cycloalkyl, phenyl and benzyl being optionally substituted by halo, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ or R⁹;
R³ is H, C₁-C₆ alkyl, C₃-C₇ cycloalkyl, phenyl, benzyl, halo, -CN, -OR⁷, -CO₂R⁵, -CONR⁵R⁵, R⁸ or R⁹, said C₁-C₆ alkyl, C₃-C₇ cycloalkyl, phenyl and benzyl being optionally substituted by halo, -CN, . -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ or R⁹;
R⁴ is phenyl, naphthyl or pyridyl, each being optionally substituted by R⁸, halo, -CN, C₁-C₆ alkyl, C₁-C₆ haloalkyl, C₃-C₇ cycloalkyl, C₁-C₆ alkoxy, -CONR⁵R⁵, OR¹¹, SOₓR⁶, O-(C₁-C₆ alkylene)-CONR⁵R⁵, O-(C₁-C₆ alkylene)-NR⁵R⁵, or O-(C₁-C₆ alkylene)-OR⁶;
each R⁵ is independently either H, C₁-C₆ alkyl or C₃-C₇ cycloalkyl or, when two R⁵ groups are attached to the same nitrogen atom, those two groups taken together with the nitrogen atom to which they are attached represent azetidinyl, pyrrolidinyl, piperidinyl, homopiperidinyl, piperazinyl, homopiperazinyl or morpholinyl, said azetidinyl, pyrrolidinyl, piperidinyl, homopiperidinyl, piperazinyl, homopiperazinyl and morpholinyl being optionally substituted by C₁-C₈ alkyl or C₃-C₇ cycloalkyl;
each R⁶ is independently either H, C₁-C₆ alkyl or C₃-C₇ cycloalkyl;
R⁷ is C₁-C₆ alkyl or C₃-C₇ cycloalkyl;
R⁸ is a five or six-membered, aromatic heterocyclic group containing (i) from 1 to 4 nitrogen heteroatom(s) or (ii) 1 or 2 nitrogen heteroatom(s) and 1 oxygen or 1 sulphur heteroatom or (iii) 1 or 2 oxygen or sulphur heteroatom(s), said heterocyclic group being optionally substituted by halo, oxo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, fluoro(C₁-C₆)alkyl or C₃-C₇ cycloalkyl;
R⁹ is a four to seven-membered, saturated or partially unsaturated heterocyclic group containing (i) 1 or 2 nitrogen heteroatom(s) or (ii) 1 nitrogen heteroatom and 1 oxygen or 1 sulphur heteroatom or (iii) 1 oxygen or sulphur heteroatom, said heterocyclic group being optionally substituted by oxo, C₁-C₆ alkyl, C₃-C₇cycloalkyl, -SO₂R⁵, -CONR⁵R⁵, -COOR⁵, -CO-(C₁-C₆ alkylene)-OR⁵ or -COR⁵ and optionally substituted on a carbon atom which is not adjacent to a heteroatom by halo, -OR⁵, -NR⁵R⁵, -NR⁵COR⁵, -NR⁵COOR⁵, -NR⁵CONR⁵R⁵, -NR⁵SO₂R⁵ or -CN;
R¹⁰ is C₁-C₆ alkyl substituted by R⁸, R⁹, -OR⁵, -CONR⁵R⁵, -NR⁵COR⁵ or -NR⁵R⁵;
R¹¹ is phenyl optionally substituted by halo, -CN, -COR⁵, -CONR⁵R⁵, -SO₂NR⁵R⁵, -NR⁵SO₂R⁵, -OR⁵, -NR⁵R⁵, -(C₁-C₆ alkylene)-NR⁵R⁵, C₁-C₆ alkyl, halo(C₁-C₆)alkyl or C₃-C₇ cycloalkyl; and
x and y are independently 0, 1 or 2.

2. A pharmaceutical composition including a compound of formula (I) according to claim 1 or a pharmaceutically acceptable salt or solvate thereof together with one or more pharmaceutically acceptable excipients, diluents or carriers.

3. A pharmaceutical composition according to claim 2 including one or more additional therapeutic agents.

4. A compound of formula (I) according to claim 1 or a pharmaceutically acceptable salt or solvate thereof, or a pharmaceutical composition according to claim 2 or 3, for use as a medicament.

5. A compound of formula (I) according to claim 1 or a pharmaceutically acceptable salt or solvate thereof, or a pharmaceutical composition according to claim 2 or 3, for use as a reverse transcriptase inhibitor or modulator.

6. A compound of formula (I) according to claim 1 or a pharmaceutically acceptable salt or solvate thereof, or a pharmaceutical composition according to claim 2 or 3, for use in the treatment of an HIV or genetically-related retroviral infection, or a resulting acquired immune deficiency syndrome (AIDS).

7. Use of a compound of formula (I) according to claim 1 or a pharmaceutically acceptable salt or solvate thereof, or a pharmaceutical composition according to claim 2 or 3, for the manufacture of a medicament having reverse transcriptase inhibitory or modulating activity.

8. Use of a compound of formula (I) according to claim 1 or a pharmaceutically acceptable salt or solvate thereof, or a pharmaceutical composition according to claim 2 or 3, for the manufacture of a medicament for the treatment of an HIV or genetically-related retroviral infection, or a resulting acquired immune deficiency syndrome (AIDS).

9. A process for preparing the compound of formula (I) according to claim 1 or a pharmaceutically acceptable salt or solvate thereof, which comprises:
(A) reaction of a compound of formula (V) 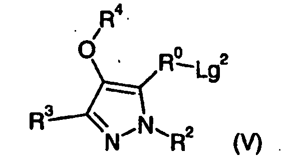 with an alcohol of formula (IV), R¹-OH (IV), under conventional conditions;
(B) reaction of an alcohol of formula (III) 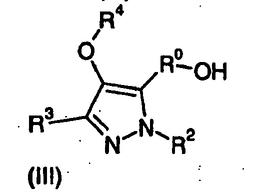 with a compound of formula (II), Lg-R¹ (II), under conventional conditions;
(C) reaction of a compound of formula (III) with an alcohol of formula (IV) under dehydrating conditions;
(D) for the preparation of a compound of formula (I) in which R³ is halo, halogenating a compound of formula (X) 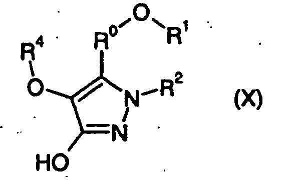 under conventional conditions;
(E) interconversion of a compound of formula (I) into another compound of formula (I); or
(F) deprotecting a protected derivative of compound of formula (I); and optionally converting a compound of formula (I) prepared by any one of processes (A) to (F) into pharmaceutically acceptable salt or solvate thereof, wherein R⁰ to R⁴ are as previously defined for a compound of formula (I), and Lg and Lg² are leaving groups.

10. A compound of formulae (V) or (X) 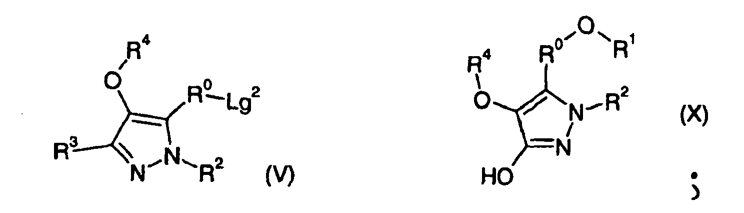 wherein Lg² is a sulphonic ester group and R⁰ to R⁴ are as previously defined for a compound of formula (I).

## Patentansprüche

1. Verbindung der Formel (I) 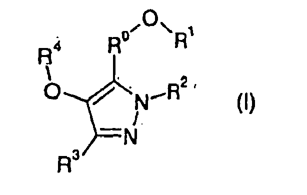 oder ein pharmazeutisch verträgliches Salz oder Solvat davon, worin:
R⁰ nicht vorhanden ist oder C₁-C₆-Alkylen ist;
R¹ Phenyl ist, substituiert durch -SO_{y}R⁵, (C₁-C₆-Alkylen)-SO_{y}R⁵, -SO_{y}CF₃, -(C₁-C₆-Alkylen)-SO_{y}CF₃, -CO₂R⁵, -(C₀-C₆-Alkylen)-CO₂R⁵, OCF₃, eine fünf- oder sechsgliedrige aromatische heterocyclische Gruppe, enthaltend (i) 1 bis 4 Stickstoffheteroatom(e) oder (ii) 1 oder 2 Stickstoffheteroatom(e) und 1 Sauerstoff- oder 1 Schwefelheteroatom (wobei die genannte heterocyclische Gruppe gegebenenfalls substituiert ist durch Halogen, Oxo, -CN, -COR⁵, -CO₂R⁵, -CONR⁵R⁵, -SO_{y}R⁵, -SO_{y}CF₃, -SO₂NR⁵R⁵, -NR⁵SO₂R⁵, -OR⁵, -OCF₃, -NR⁵R⁵, -(C₁-C₆-Alkylen)-NR⁵R⁵, C₁-C₆-Alkyl, Fluor (C₁-C₆)alkyl oder C₃-C₇-(Cycloalkyl); wobei das genannte Phenyl gegebenenfalls zusätzlich substituiert ist durch Halogen, -CN, -COR⁵, -CONR⁵R⁵, -SO₂NR⁵R⁵, -NR⁵SO₂R⁵, -OR⁵, -NR⁵R⁵, -(C₁-C₆-Alkylen)-NR⁵R⁵, C₁-C₆-Alkyl, Halogen(C₁-C₆)alkyl oder C₃-C₇-Cycloalkyl;
R² ist H, C₁-C₆-Alkyl, C₃-C₆-Alkenyl, C₃-C₆-Alkinyl, C₃-C₇-Cycloalkyl, C₃-C-₇-Cycloalkenyl, Phenyl, Benzyl, R⁸ oder R⁹, wobei das genannte C₁-C₆-Alkyl, C₃-C₇-Cycloalkyl, Phenyl und Benzyl gegebenenfalls substituiert sind durch Halogen, -OR⁵, -OR¹⁰, -CN, -CO₂R⁷, -OCONR⁵R⁵, -CONR⁵R⁵, -C (=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ oder R⁹;
R³ ist H, C₁-C₆-Alkyl, C₃-C₇-Cycloalkyl, Phenyl, Benzyl, Halogen, -CN, -OR⁷, -CO₂R⁵, -CONR⁵R⁵, R⁸ oder R⁹, das genannte C₁-C₆-Alkyl, C₃-C₇-Cycloalkyl, das Phenyl und Benzyl sind gegebenenfalls substituiert durch Halogen -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ oder R⁹;
R⁴ ist Phenyl, Naphthyl oder Pyridyl, jeweils gegebenenfalls substituiert durch R⁸, Halogen, -CN, C₁-C₆-Alkyl, C₁-C₆-Halogenalkyl, C₃-C₇-Cycloalkyl, C₁-C₆-Alkoxy, -CONR⁵R⁵, OR¹¹, SOₓR⁶, 0- (C₁-C₆-Alkylen) -CONR⁵R⁵, 0- (C₁-C₆-Alkylen) -NR⁵R⁵ oder O-(C₁-C₈-Alkylen)-OR⁶;
R⁵ ist jeweils unabhängig entweder H, C₁-C₆-Alkyl oder C₃-C₇-Cycloalkyl, oder, wenn zwei R⁵-Gruppen mit dem selben Stickstoffatom verbunden sind, verkörpern diese beide Gruppen zusammengenommen mit dem Stickstoffatom, mit dem sie verbunden sind, Azetidinyl, Pyrrolidinyl, Piperidinyl, Homopiperidinyl, Piperazinyl, Homopiperazinyl oder Morpholinyl, wobei das genannte Azetidinyl, Pyrrolidinyl, Piperidinyl, Homopiperidinyl, Piperazinyl, Homopiperazinyl und Morpholinyl gegebenenfalls substituiert sind durch C₁-C₆-Alkyl oder C₃-C₇-Cycloalkyl;
jedes R⁶ ist unabhängig entweder H, C₁-C₆-Alkyl oder C₃-C₇-Cycloalkyl;
R⁷ ist C₁-C₆-Alkyl oder C₃-C₇-Cycloalkyl;
R⁸ ist eine fünf- oder sechsgliedrige aromatische heterocyclische Gruppe, enthaltend (i) 1 bis 4 Stickstoffheteroatom(e) oder (ii) 1 oder 2 Stickstoffheteroatom(e) und 1 Sauerstoff- oder 1 Schwefelheteroatom oder (iii) 1 oder 2 Sauerstoff- oder Schwefelheteroatom(e), wobei die genannte heterocyclische Gruppe gegebenenfalls substituiert ist durch Halogen, Oxo, -CN, -COR⁵, -CONR⁵R⁵, -SO₂NR⁵R⁵, -NR⁵SO₂R⁵, -OR⁵, -NR⁵R⁵, -(C₁-C₆-Alkylen) -NR⁵R⁵, C₁-C₆-Alkyl, Fluor(C₁-C₆)Alkyl oder C₃-C₇-Cycloalkyl;
R⁹ ist eine vier- bis siebengliedrige gesättigte oder teilweise ungesättigte heterocyclische Gruppe, enthaltend (i) 1 oder 2 Stickstoffheteroatom(e) oder (ii) 1 Stickstoffheteroatom und ein 1 Sauerststoff- oder ein 1 Schwefelheteroatom oder (iii) ein Sauerstoff- oder Schwefelheteroatom, wobei die genannte heterocyclische Gruppe gegebenenfalls substituiert ist durch Oxo, C₁-C₆-Alkyl, C₃-C₇-Cycloalkyl, -SO₂R⁵, -CONR⁵R⁵, -COOR⁵, -CO-(C₁-C₆-Alkylen)-OR⁵ oder -COR⁵ und gegebenenfalls substituiert ist an einem Kohlenstoffatom, das einem Heteroatom nicht benachbart ist, durch Halogen, -OR⁵, -NR⁵R⁵, -NR⁵COR⁵, -NR⁵COOR⁵, -NR⁵CONR⁵R⁵, -NR⁵SO₂R⁵ oder -CN;
R¹⁰ ist C₁-C₆-Alkyl, substituiert durch R⁸, R⁹, -OR⁵, -CONR⁵R⁵, -NR⁵COR⁵ oder -NR⁵R⁵;
R¹¹ ist Phenyl, gegebenenfalls substituiert durch Halogen, -CN, -COR⁵, -CONR⁵R⁵, -SO₂NR⁵R⁵, -NR⁵SO₂R⁵, -OR⁵, -NR⁵R⁵, -(C₁-C₆-Alkylen) -NR⁵R⁵, C₁-C₆-Alkyl, Halogen(C₁-C₆)alkyl oder C₃-C₇-Cycloalkyl; und
x und y sind unabhängig 0, 1 oder 2.

2. Pharmazeutische Zusammensetzung, umfassend eine Verbindung der Formel (I) gemäß Anspruch 1 oder ein pharmazeutisch verträgliches Salz oder Solvat davon, zusammen mit einem oder mehreren pharmazeutisch verträglichen Exzipientien, Verdünnungsmitteln oder Carriern.

3. Pharmazeutische Zusammensetzung gemäß Anspruch 2, umfassend ein oder mehrere zusätzliche(s) therapeutische(s) Mittel.

4. Verbindung der Formel (I) gemäß Anspruch 1 oder pharmazeutisch verträgliches Salz oder Solvat davon oder pharmazeutische Zusammensetzung gemäß Anspruch 2 oder 3 zur Verwendung als Medikament.

5. Verbindung der Formel (I) gemäß Anspruch 1 oder pharmazeutisch verträgliches Salz oder Solvat davon oder pharmazeutische Zusammensetzung gemäß Anspruch 2 oder 3 zur Verwendung als Reverse-Transkriptase-Inhibitor oder -Modulator.

6. Verbindung der Formel (I) gemäß Anspruch 1 oder pharmazeutisch verträgliches Salz oder Solvat davon oder pharmazeutische Zusammensetzung gemäß Anspruch 2 oder 3 zur Verwendung in der Behandlung einer HIV- oder genetisch verwandten retroviralen Infektion oder des resultierenden erworbenen Immunschwächesyndroms (AIDS).

7. Verwendung einer Verbindung der Formel (I) gemäß Anspruch 1 oder eines pharmazeutisch verträglichen Salzes oder Solvats davon oder einer pharmazeutischen Zusammensetzung nach Anspruch 2 oder 3 zur Herstellung eines Medikaments, das Reverse-Transkriptase-inhibierende oder -modulierende Aktivität besitzt.

8. Verwendung einer Verbindung der Formel (I) gemäß Anspruch 1 oder eines pharmazeutisch verträglichen Salzes oder Solvats davon oder einer pharmazeutischen Zusammensetzung gemäß Anspruch 2 oder 3 zur Herstellung eines Medikaments zur Behandlung von HIV oder einer genetisch verwandten retroviralen Infektion oder eines resultierenden erworbenen Immunschwächesyndroms (AIDS).

9. Verfahren zur Herstellung der Verbindung der Formel (I) gemäß Anspruch 1 oder eines pharmazeutisch verträglichen Salzes oder Solvats davon, das umfasst:
(A) Reaktion einer Verbindung der Formel (V) 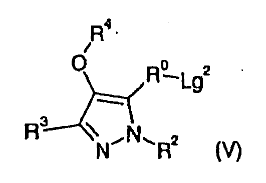 mit einem Alkohol der Formel (IV), R¹-OH (IV), unter herkömmlichen Bedingungen;
(B) Reaktion eines Alkohols der Formel (III) 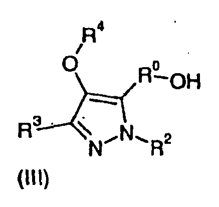 mit einer Verbindung der Formel (II), Lg-R¹ (II), unter herkömmlichen Bedingungen;
(C) Reaktion einer Verbindung der Formel (III) mit einem Alkohol der Formel (IV) unter dehydratisierenden Bedingungen;
(D) zur Herstellung einer Verbindung der Formel (I), worin R³ Halogen ist, Halogenieren einer Verbindung der Formel (X) 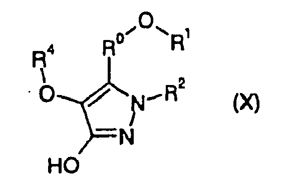 unter herkömmlichen Bedingungen;
(E) Umwandlung ("Interconversion") einer Verbindung der Formel (I) in eine andere Verbindung der Formel (I); oder
(F) Entschützen eines geschützten Derivats der Verbindung der Formel (I) und gegebenenfalls Umwandeln einer Verbindung der Formel (I), hergestellt durch ein beliebiges der Verfahren (A) bis (F), in ein pharmazeutisch verträgliches Salz oder Solvat davon, wobei R⁰ bis R⁴ wie vorstehend definiert sind für eine Verbindung der Formel (I), und Lg und Lg² sind Abgangsgruppen.

10. Verbindung der Formeln (V) oder (X) 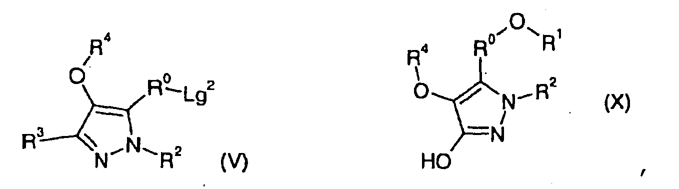 wobei Lg² eine Sulfonestergruppe ist und R⁰ bis R⁴ wie vorstehend definiert sind für eine Verbindung der Formel (I).

## Revendications

1. Composé de formule (I) 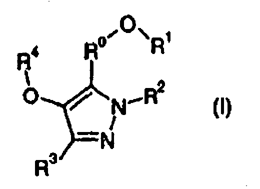 ou sel ou solvate pharmaceutiquement acceptable correspondant, dans lequel :
R⁰ est absent ou représente alkylène en C₁-C₆ ;
R¹ représente phényle substitué par -SO_{y}R⁵, (alkylène en C₁-C₆)-SO_{y}R⁵, -SO_{y}CF₃, -(alkylène en C₁-C₆)-SO_{y}CF₃, -CO₂R⁵, -(alkylène en C₀-C₆)-CO₂R⁵, OCF₃, un groupe hétérocyclique aromatique ayant cinq ou six éléments et contenant (i) de 1 à 4 hétéroatome(s) d'azote ou (ii) 1 ou 2 hétéroatome(s) d'azote et 1 hétéroatome d'oxygène ou de soufre (ledit groupe hétérocyclique étant facultativement substitué par halogéno, oxo, -CN, -COR⁵, -CO₂R⁵, -CONR⁵R⁵, -SO_{y}R⁵, -SO_{y}CF₃, -SO₂NR⁵R⁵, -NR⁵SO₂R⁵, -OR⁵, -OCF₃, -NR⁵R⁵, -(alkylène en C₁-C₆)-NR⁵R⁵, alkyle en C₁-C₆, fluoro(alkyle en C₁-C₆) ou cycloalkyle en C₃-C₇; ledit groupe phényle étant, en outre, facultativement substitué par halogéno, -CN, -COR⁵, -CONR⁵R⁵, -SO₂NR⁵R⁵, -NR⁵SO₂R⁵, -OR⁵, -NR⁵R⁵, -(alkylène en C₁-C₆)-NR⁵R⁵, alkyle en C₁-C₆, halogéno(alkyle en C₁-C₆) ou cycloalkyle en C₃-C₇ ;
R² représente H, alkyle en C₁-C₆, alkényle en C₃-C₆, alkynyle en C₃-C₆, cycloalkyle en C₃-C₇, cycloalkényle en C₃-C₇, phényle, benzyle, R⁸ ou R⁹, lesdits groupes alkyle en C₁-C₆, cycloalkyle en C₃-C₇, phényle et benzyle étant facultativement substitués par halogéno, -OR⁵, -OR¹⁰, -CN, -CO₂R⁷, -OCONR⁵R⁵, -CONR⁵R⁵, -C(=NR⁵)NR⁵OR⁵, -CONR⁵NR⁵R⁵, -NR⁶R⁶, -NR⁵R¹⁰, -NR⁵COR⁵, -NR⁵COR⁸, -NR⁵COR¹⁰, -NR⁵CO₂R⁵, -NR⁵CONR⁵R⁵, -SO₂NR⁵R⁵, -NR⁵SO₂R⁵, -NR⁵SO₂NR⁵R⁵, R⁸ ou R⁹ ;
R³ représente H, alkyle en C₁-C₆, cycloalkyle en C₃-C₇, phényle, benzyle, halogéno, -CN, -OR⁷, -CO₂R⁵, -CONR⁵R⁵, R⁸ ou R⁹, lesdits groupes alkyle en C₁-C₆, cycloalkyle en C₃-C₇, phényle et benzyle étant facultativement substitués par halogéno, -CN, -OR⁵, -CO₂R⁵, -CONR⁵R⁵, -OCONR⁵R⁵, -NR⁵CO₂R⁵, -NR⁶R⁶, -NR⁵COR⁵, -SO₂NR⁵R⁵, -NR⁵CONR⁵R⁵, -NR⁵SO₂R⁵, R⁸ ou R⁹ ;
R⁴ représente phényle, naphtyle ou pyridyle, chacun d'entre eux étant facultativement substitué par R⁸, halogéno, -CN, alkyle en C₁-C₆, halogéno (alkyle en C₁-C₆), cycloalkyle en C₃-C₇, alcoxy en C₁-C₆, -CONR⁵R⁵, -OR¹¹, -SOₓR⁶, O-(alkylène en C₁-C₆) -CONR⁵R⁵, O-(alkylène en C₁-C₆)-NR⁵R⁵ ou O-(alkylène en C₁-C₆) -OR⁶ ;
chaque R⁵ représentant indépendamment H, alkyle en C₁-C₆ ou cycloalkyle en C₃-C₇ ou, lorsque deux groupes R⁵ sont fixés au même atome d'azote, ces deux groupes, pris ensemble avec l'atome d'azote auquel ils sont liés, représentant azétidinyle, pyrrolidinyle, pipéridinyle, homopipéridinyle, pipérazinyle, homopipérazinyle ou morpholinyle, lesdits groupes azétidinyle, pyrrolidinyle, pipéridinyle, homopipéridinyle, pipérazinyle, homopipérazinyle et morpholinyle étant facultativement substitués par alkyle en C₁-C₆ ou cycloalkyle en C₃-C₇ ;
chaque R⁶ représentant indépendamment H, alkyle en C₁-C₆ ou cycloalkyle en C₃-C₇ ;
R⁷ représentant alkyle en C₁-C₆ ou cycloalkyle en C₃-C₇ ;
R⁸ étant un groupe hétérocyclique aromatique ayant cinq ou six éléments et contenant (i) de 1 à 4 hétéroatome(s) d'azote ou (ii) 1 ou 2 hétéroatome(s) d'azote et 1 hétéroatome d'oxygène ou de soufre, ou (iii) 1 ou 2 hétéroatome(s) d'oxygène ou de soufre, ledit groupe hétérocyclique étant facultativement substitué par halogéno, oxo, -CN, -COR⁵, -CONR⁵R⁵, -SO₂NR⁵R⁵, -NR⁵SO₂R⁵, -OR⁵, -NR⁵R⁵, -(alkylène en C₁-C₆) -NR⁵R⁵, alkyle en C₁-C₆, fluoro (alkyle en C₁-C₆) ou cycloalkyle en C₃-C₇ ;
R⁹ étant un groupe hétérocyclique saturé ou partiellement insaturé ayant de quatre à sept éléments et contenant (i) 1 ou 2 hétéroatome(s) d'azote ou (ii) 1 hétéroatome d'azote et 1 hétéroatome d'oxygène ou de soufre, ou (iii) 1 hétéroatome d'oxygène ou de soufre, ledit groupe hétérocyclique étant facultativement substitué par oxo, alkyle en C₁-C₆, cycloalkyle en C₃-C₇, -SO₂R⁵, -CONR⁵R⁵, -COOR⁵, -CO-(alkylène en C₁-C₆)-OR⁵ ou -COR⁵, et facultativement substitué sur un atome de carbone qui n'est pas adjacent à un hétéroatome par halogéno, -OR⁵, -NR⁵R⁵, -NR⁵COR⁵, -NR⁵COOR⁵, -NR⁵CONR⁵R⁵, -NR⁵SO₂R⁵ ou -CN ;
R¹⁰ représentant alkyle en C₁-C₆ substitué par R⁸, R⁹, -OR⁵, -CONR⁵R⁵, -NR⁵COR⁵ ou -NR⁵R⁵ ;
R¹¹ représentant phényle facultativement substitué par halogéno, -CN, -COR⁵, -CONR⁵R⁵, -SO₂NR⁵R⁵, -NR⁵SO₂R⁵, -OR⁵, -NR⁵R⁵, -(alkylène en C₁-C₆) -NR⁵R⁵, alkyle en C₁-C₆, halogéno(alkyle en C₁-C₆) ou cycloalkyle en C₃-C₇ ; et
x et y représentant indépendamment 0, 1 ou 2.

2. Composition pharmaceutique incluant un composé de formule (I) selon la revendication 1, ou un sel ou solvate pharmaceutiquement acceptable correspondant, en même temps qu'un ou plusieurs excipients, diluants ou supports pharmaceutiquement acceptables.

3. Composition pharmaceutique selon la revendication 2, incluant un ou plusieurs agents thérapeutiques supplémentaires.

4. Composé de formule (I) selon la revendication 1, ou sel ou solvate pharmaceutiquement acceptable correspondant, ou composition pharmaceutique selon la revendication 2 ou 3, pour une utilisation en tant que médicament.

5. Composé de formule (I) selon la revendication 1, ou sel ou solvate pharmaceutiquement acceptable correspondant, ou composition pharmaceutique selon la revendication 2 ou 3, pour une utilisation en tant qu'inhibiteur ou modulateur de la transcriptase inverse.

6. Composé de formule (I) selon la revendication 1, ou sel ou solvate pharmaceutiquement acceptable correspondant, ou composition pharmaceutique selon la revendication 2 ou 3, pour une utilisation dans le traitement d'une infection rétrovirale à VIH, ou à virus génétiquement proche, ou d'un syndrome d'immunodéficience acquise (SIDA) en résultant.

7. Utilisation d'un composé de formule (I) selon la revendication 1, ou sel ou solvate pharmaceutiquement acceptable correspondant, ou composition pharmaceutique selon la revendication 2 ou 3, pour la fabrication d'un médicament ayant une activité inhibitrice ou modulatrice envers la transcriptase inverse.

8. Utilisation d'un composé de formule (I) selon la revendication 1, ou sel ou solvate pharmaceutiquement acceptable correspondant, ou composition pharmaceutique selon la revendication 2 ou 3, pour la fabrication d'un médicament pour le traitement d'une infection rétrovirale à VIH, ou à virus génétiquement proche, ou d'un syndrome d'immunodéficience acquise (SIDA) en résultant.

9. Procédé de préparation du composé de formule (I) selon la revendication 1, ou d'un sel ou solvate pharmaceutiquement acceptable correspondant, qui comprend :
(A) la réaction d'un composé de formule (V) 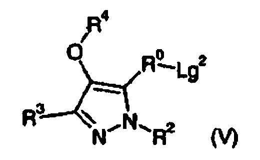 avec un alcool de formule (IV), R¹-OH (IV), dans des conditions classiques ;
(B) la réaction d'un alcool de formule (III) 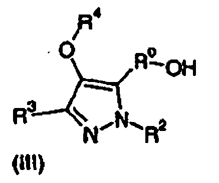 avec un composé de formule (II), Lg-R¹ (II), dans des conditions classiques ;
(C) la réaction d'un composé de formule (III) avec un alcool de formule (IV), dans des conditions de déshydratation ;
(D) pour la préparation d'un composé de formule (I) dans lequel R³ représente halogéno, l'halogénation d'un composé de formule (X) 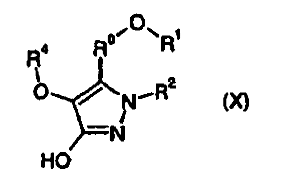 dans des conditions classiques ;
(E) l'interconversion d'un composé de formule (I) en un autre composé de formule (I) ; ou
(F) la déprotection d'un dérivé protégé d'un composé de formule (I) ; et la transformation facultative d'un composé de formule (I), préparé avec l'un quelconque des procédés (A) à (F), en un sel ou solvate pharmaceutiquement acceptable correspondant, les groupes R⁰ à R⁴ étant tels que définis précédemment pour un composé de formule (I), et Lg et Lg² étant des groupes labiles.

10. Composé de formule (V) ou (X) 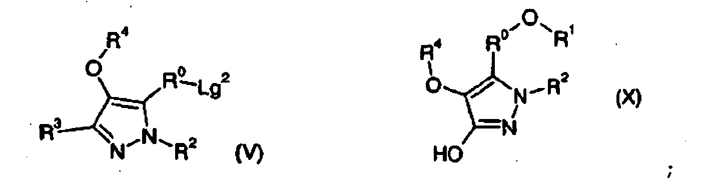 dans lequel Lg² est un groupe ester sulfonique et les groupes R⁰ à R⁴ sont tels que définis précédemment pour un composé de formule (I).
